# DEMANDE DE BREVET EUROPEEN

(11) **EP 0 864 322 A1**
(43) Date de publication de la demande: **16.09.1998**
(21) Numéro de dépôt: 98400547.0
(22) Date de dépôt: 09.03.1998
(51) Int. Cl.: A61K 7/48, A61K 7/035

(54) **Procédé de préparation d'une composition cosmétique solide à base de plâtre et composition cosmétique ainsi obtenue**

(30) Priorité: 13.03.1997 FR 9703168
(71) Demandeur: L'OREAL, 75008 Paris (FR)
(72) Inventeur: Bodelin-Lecomte, Sophie, 92170 Vanves (FR); Le Gars, Guénola, 75013 Paris (FR)
(74) Mandataire: Lhoste, Catherine

(57) **Abrégé**

L'invention concerne un procédé de préparation d'une composition, notamment cosmétique, solide à base de plâtre comprenant les étapes suivantes :
- on prépare un mélange pulvérulent comprenant au moins du sulfate de calcium hémihydraté sous forme de poudre,
- on ajoute au mélange pulvérulent un phase aqueuse,
- on malaxe le mélange pulvérulent et la phase aqueuse de façon à obtenir une mélange coulable,
- on verse le mélange coulable dans un moule,
- on laisse durcir le mélange par hydratation du sulfate de calcium hémihydraté en sulfate de calcium dihydraté,
caractérisé par le fait que l'on ajoute dans le mélange pulvérulent, avant d'ajouter la phase aqueuse, un système conservateur liquide non aqueux.

L'invention concerne aussi une composition, notamment cosmétique, susceptible d'être obtenue par le procédé.

## Description

La présente invention concerne un procédé de fabrication d'une composition solide à l'aide de plâtre et une composition, notamment cosmétique, obtenue par ce procédé.

Il est connu de fabriquer des compositions cosmétiques sous forme solide à l'aide de plâtre. Ces compositions cosmétiques peuvent être notamment des fards à paupières, des fards à joues, des poudres pour le visage ou pour le corps. Elles peuvent se présenter sous forme de stick, de crayons ou de pains. L'utilisation du plâtre pour la fabrication de ces compositions solides est, par exemple, décrit dans le brevet US-A-4724138. Selon les procédés connus, une phase pulvérulente contenant du plâtre est mélangée avec une phase aqueuse pour obtenir un pâte que l'on met en forme par moulage, la réaction d'hydratation du plâtre (sulfate de calcium hémihydraté) en sulfate de calcium dihydraté provoquant la solidification de la composition. L'utilisation de plâtre comme agent de solidification est avantageuse car elle permet de remplacer l'opération de compactage, habituellement nécessaire pour obtenir une composition sous forme solide à partir de produits pulvérulents, par une opération de moulage, dont la mise en oeuvre est plus simple, moins coûteuse et permet d'obtenir des formes plus diverses.

Par ailleurs, il est d'usage courant d'ajouter dans les compositions cosmétiques des conservateurs dans le but de conférer à la composition une bonne conservation, notamment pour empêcher la prolifération de micro-organismes dans la composition.
Il existe différentes manières d'incorporer les conservateurs dans les compositions à base de plâtre. Les conservateurs peuvent être ajoutés soit à la phase pulvérulente comprenant le plâtre, soit à la phase aqueuse avant de mélanger ces deux phases pour former la pâte coulable. C'est ainsi que dans le brevet US-A-4724138, le para hydroxy benzoate de méthyle est ajouté dans la phase de poudre. La demande WO 86/00798, quant à elle, propose d'incorporer les conservateurs dans la phase aqueuse.
La demanderesse a constaté que lorsque les conservateurs sont ajoutés dans la phase aqueuse, la concentration de ces agents conservateurs n'est pas homogène dans la composition finale obtenue après évaporation de l'eau. En effet, il existe dans la composition finale un gradient de concentration des conservateurs. Les conservateurs se trouvent alors concentrés vers la surface d'évaporation de la composition. La protection de la composition contre le développement de micro-organismes n'est donc pas identique en toute zone de la composition. En outre, lorsque la composition est un produit de maquillage, par exemple un fard à joues, l'application sur la peau de la partie de la composition renfermant la concentration la plus élevée en conservateur peut engendrer des problèmes de tolérance de la composition sur la peau : des irritations ou des picotements de la peau peuvent alors être provoqués par une concentration trop élevée en conservateurs dans la composition appliquée.

On a également constaté que le gradient de concentration des conservateurs se forme aussi lorsque l'on ajoute un conservateur sous forme de poudre, quelle que soit la phase dans laquelle il est introduit.

La présente invention a pour but de pallier ces inconvénients et propose un procédé de préparation d'une composition notamment cosmétique qui permet d'obtenir une répartition homogène des conservateurs dans la composition finale. Par répartition homogène, on entend que la concentration des conservateurs est sensiblement constante en toute partie de la composition finale.

La demanderesse a découvert de façon inattendue et surprenante que l'emploi d'un système conservateur liquide permettait d'obtenir une composition avec une répartition homogène du ou des conservateurs.

La présente invention a donc pour objet un procédé de préparation d'une composition solide à base de plâtre comprenant les étapes suivantes :
- on prépare un mélange pulvérulent comprenant au moins du sulfate de calcium hémihydraté sous forme de poudre,
- on ajoute au mélange pulvérulent un phase aqueuse,
- on malaxe le mélange pulvérulent et la phase aqueuse de façon à obtenir une mélange coulable,
- on verse le mélange coulable dans un moule,
- on laisse durcir le mélange par hydratation du sulfate de calcium hémihydraté en sulfate de calcium dihydraté,
caractérisé par le fait que l'on ajoute dans le mélange pulvérulent, avant d'ajouter la phase aqueuse, un système conservateur liquide non aqueux comprenant au moins un conservateur ayant une solubilité dans l'eau inférieure ou égale à 0,4 % à 25°C (pourcentage poids/poids), ledit système conservateur liquide ayant une viscosité inférieure ou égale à 4,5 Pa. s.

L'invention concerne aussi une composition, notamment cosmétique, susceptible d'être obtenue par le procédé selon l'invention. La composition ainsi obtenue convient parfaitement pour le maquillage. Elle peut être utilisée comme fard à joue, fard à paupières, ou poudre pour le visage.

L'invention concerne également une composition cosmétique solide à base de plâtre comprenant un système conservateur réparti de façon homogène dans ladite composition, ledit système conservateur étant, hors composition, sous la forme d'un liquide non aqueux comprenant au moins un conservateur ayant une solubilité dans l'eau inférieure ou égale à 0,5 % à 25 °C, ledit système conservateur liquide ayant une viscosité inférieure ou égale à 4,5 Pa.s à 25 °C.

Par conservateur, on entend tout composé ayant une action antimicrobienne et/ou antioxydante. On utilise de préférence un conservateur ayant une activité antimicrobienne.

Par système conservateur liquide non aqueux, on entend selon la présente demande soit un ou plusieurs conservateurs dont le mélange est liquide à 25°C, soit un ou plusieurs conservateurs, liquides ou solides, en mélange avec un solvant non aqueux, le mélange étant liquide à 25°C.

De préférence, le système conservateur liquide selon l'invention présente une viscosité inférieure à 3 Pa.s à 25 °C et plus préférentiellement inférieure à 1,5 Pa.s à 25 °C.

La viscosité du conservateur liquide selon l'invention peut par exemple être mesurée sur un viscosimètre Rhéomat RM115 de chez CONTRAVES, sur mobile adéquat (par exemple MSDIN 108, 114, 125), à un taux de cisaillement 221,3 s⁻¹, après 10 minutes.

Il est nécessaire que le système conservateur ne soit pas aqueux, car l'addition du conservateur dans une phase aqueuse ne permet pas d'obtenir une répartition homogène du conservateur dans la composition finale.

Le solvant non aqueux liquide selon l'invention est tout composé liquide compatible avec le conservateur, le conservateur ou le mélange de conservateurs pouvant être soit dissous, en totalité ou en partie, soit dispersé de manière homogène dans ledit solvant au moins extemporanément, c'est-à-dire que le conservateur ou le mélange de conservateurs est bien réparti dans le solvant au moment de la préparation du mélange. De préférence, en particulier lorsque le conservateur ou le mélange de conservateurs est sous forme de poudre, le conservateur ou le mélange de conservateurs est solubilisé, en totalité ou en partie, dans le solvant. Ainsi, le mélange de conservateur et de solvant doit présenter la viscosité requise selon l'invention. Le solvant du système conservateur est choisi de telle sorte qu'il ne nuise pas à l'activité antimicrobienne et/ou antioxydante du conservateur.

Avantageusement, on peut utiliser un solvant du conservateur permettant d'obtenir une solution du conservateur dans le solvant (dissolution totale du conservateur dans le solvant). Le conservateur mélangé audit solvant n'est pas nécessairement liquide à 25°C. Il peut notamment se présenter sous forme de poudre mais sa solubilité dans l'eau à 25°C doit être inférieure à 0,5 % (pourcentage poids/poids), de préférence inférieure à 0,4 % et plus préférentiellement inférieure à 0,35 %.

Avantageusement, on peut utiliser un système conservateur comprenant un mélange conservateur/solvant dans un rapport pondéral conservateur/solvant allant environ de 10/90 à 90/10, et de préférence de 20/80 à 40/60.

Comme conservateur liquide, on peut citer le mélange de para hydroxy benzoate d'isopropyle, de para hydroxy benzoate d'isobutyle et de para hydroxy benzoate de n-butyle (dans les proportions en poids respectives de 40/30/30) vendu sous la dénomination "LIQUAPAR OIL" par la société ISP. Ce mélange de conservateur est insoluble dans l'eau (solubilité dans l'eau inférieure à 0,1 % à 25 °C). Ce mélange de conservateur peut être mélangé à un solvant compatible avant d'être ajouté au mélange pulvérulent. Il peut également être associé à un autre conservateur tout en respectant les caractéristiques de l'invention.

Comme conservateur non liquide ayant une solubilité dans l'eau inférieure à 0,4 % à 25 °C, on peut citer le para hydroxy benzoate de méthyle (solubilité de 0,30 %).

Avantageusement, la teneur en conservateur incorporé dans le mélange pulvérulent peut aller de 0,01 % à 2 % en poids par rapport au poids du mélange pulvérulent, et de préférence de 0,1 % à 0,6 %.

Le sulfate de calcium hémihydraté utilisé selon l'invention peut être sous sa forme α et/ou sous sa forme β. Il peut être mélangé à au moins un agent de modification du temps de prise tel que des retardateurs comme le citrate de sodium ou des accélérateurs comme le gypse ou les sulfates de sodium.
La quantité de plâtre présent dans le mélange pulvérulent peut aller de 10 % à 70 % en poids par rapport au poids total du mélange pulvérulent, de préférence de 15 % à 35 % et plus préférentiellement de 20 % à 30 %.

Pour assurer une bonne cohésion à la composition finale, on peut ajouter à la phase pulvérulente au moins un corps gras. Comme corps gras utilisables, on peut citer les huiles minérales telles que l'huile de vaseline, les huiles d'origine animale telles que la lanoline, les huiles d'origine végétale telles que l'huile de jo-joba, les esters d'acides carboxyliques et d'alcool gras en C10-C22, les esters d'acides gras et d'alcool tel que le palmitate d'isopropyle, les alcools gras, notamment les alcools gras en en C₁₀-C₂₂ tels que l'alcool oléylique, l'alcool isostéarylique, l'octyldodécanol, les huiles de synthèse telles que les poly alpha oléfines, hydrogénées ou non, comme le polyisobutène (Parléam), les polydécènes, les huiles de silicone, notamment les huiles de silicone phénylées, les gommes de silicone ou les cires de silicone telles que les alkyldiméthicones, les huiles fluorées.

De préférence, la quantité de corps gras ajouté dans le mélange pulvérulent peut aller de 0,1 % à 20 % en poids par rapport au poids total du mélange pulvérulent, et de préférence de 0,5 % à 15 %.

Selon une première variante du procédé selon l'invention, le système conservateur peut être mélangé à au moins un corps gras avant d'être ajouté à la phase pulvérulente. En particulier, le système conservateur peut résulter du mélange du conservateur avec au moins un corps gras, à la condition que ce mélange soit homogène, c'est-à-dire que le conservateur soit bien réparti dans le corps gras.

Avantageusement, le mélange de parahydroxybenzoates (LIQUAPAR OIL) peut être mélangé dans le palmitate d'isopropyle, dans une proportion conservateur/solvant poids/poids allant de 20/80 à 40/60.

Le mélange pulvérulent peut avantageusement comprendre au moins un agent tensioactif pour faciliter le mouillage et la dispersion du mélange pulvérulent. L'agent tensioactif peut être de nature non ionique comme les esters de sorbitane polyoxyéthylénés, de nature cationique comme les sels d'ammonium quaternaires, ou de nature amphotère comme les dérivés de bétaïne.

La quantité de tensioactif introduite dans le mélange pulvérulent peut aller de 0,1 % à 10 % en poids par rapport au poids total du mélange pulvérulent.

Selon une deuxième variante du procédé selon l'invention, le tensioactif présent dans le mélange pulvérulent peut être utilisé comme solvant du système conservateur s'il permet d'obtenir un mélange compatible. On a constaté que les esters de sorbitane polyoxyéthylénés conviennent parfaitement comme solvant mélangé au conservateur selon l'invention.

Avantageusement, le mélange de parahydroxybenzoates (LIQUAPAR OIL) peut être mélangé avec le monolaurate de sorbitane polyoxyéthyléné à 20 moles d'oxyde d'éthylène vendu sous la dénomination "TWEEN 20" par la société ICI. Le para hydroxy benzoate de méthyle et le sorbate de potassium peuvent également être mélangés au "Tween 20".

Le mélange pulvérulent peut comprendre au moins une matière pulvérulente hydrophobe. Cette matière hydrophobe confère notamment de bonnes propriétés cosmétiques à la composition finale.
Par matière pulvérulente hydrophobe, on entend aussi bien des poudres hydrophobes par nature (non traitées par enrobage ou greffage chimique), que des poudres traitées par enrobage ou greffage chimique de façon à lui donner des caractéristiques hydrophobes.
Pour déterminer si, selon l'invention, une matière pulvérulente est hydrophobe, on effectue le test ci-après défini. On remplit avec 20 ml d'eau un tube à essai d'un diamètre de 20 mm. On verse 2 grammes de poudre dans le tube sans agiter et on observe le comportement de la poudre pendant 5 minutes au maximum. Si la poudre reste parfaitement en surface, elle est considérée comme "hydrophobe". Dans le cas contraire, elle est considérée comme "hydrophile".

Comme poudre hydrophobe par nature, on peut mentionner le talc (silicate de magnésium hydraté), les poudres de polymères hydrophobes, telles que la poudre des polyamides de type Nylon, par exemple celle commercialisée sous la dénomination "ORGASOL 2002 ED NAT COS" par la société ATOCHEM, la poudre de polyéthylène ("COATHYLENE HA 1681" de la société PLAST LABOR), les microsphères expansées en matériau thermoplastique ("EXPANCEL 551 DE" de la société CASCO NOBEL), les poudres polyfluorées notamment de polytétrafluoroéthylène ("MP 1400" de la société DU PONT DE NEMOURS), les poudres de silicone ("TOSPEARL" de la société TOSHIBA), les poudres de copolymères acryliques ("POLYTRAP Q5 6603 de la société DOW CHEMICAL), les poudres de polystyrène ("POLYSPHERE 3000 SP" de la société PRESPERSE), les lipoaminoacides comme la lauroyl lysine, le nitrure de bore, les savons métalliques d'acides carboxyliques en C8-C22, plus particulièrement en C12-C18, par exemple les stéarates de zinc, et de magnésium, le laurate de zinc ou le myristate de magnésium.

Les poudres traitées par enrobage ou greffage chimique peuvent être des produits pulvérulents de nature aussi bien hydrophobe qu'hydrophile, qui ont été traitées par des produits hydrophobes, parmi lesquels ont peut citer, par exemples, les silicones, les lipoaminoacides, les savons métalliques, les dérivés fluorés, les huiles minérales, la lécithine, le triisostéaroyl titanate d'isopropyle, le polyéthylène et le collagène et ses dérivés.

La quantité de matière pulvérulente hydrophobe introduite dans le mélange pulvérulent peut aller de 10 % à 60 % en poids par rapport au poids total du mélange pulvérulent, et de préférence de 20 % à 45 %.

Selon une troisième variante du procédé selon l'invention, au moins une fraction de la poudre hydrophobe est mélangée avec le système conservateur selon l'invention avant d'être ajouté à la phase pulvérulente. Le système conservateur est ainsi imprégné sur la poudre hydrophobe avant d'être incorporé à la phase pulvérulente. Cette variante du procédé selon l'invention permet d'obtenir une bonne répartition du conservateur dans la composition finale.
De préférence, comme poudre hydrophobe mélangée audit système conservateur, on peut utiliser des poudres de polyamide de type Nylon.

Avantageusement, on peut ajouter le mélange de parabène, mélangé avec le palmitate d'isopropyle ou le "Tween 20", avec la poudre de Nylon "ORGASOL 2002 ED NAT COS", puis ajouter cette poudre ainsi imprégnée dans le mélange pulvérulent comprenant le plâtre. De la même façon, le para hydroxy benzoate de méthyle et le sorbate de potassium, mélangés au "Tween 20" et avec la poudre de nylon, avant d'être incorporés dans le mélange pulvérulent.
A la place de la poudre de nylon, on peut également utiliser la poudre de copolymère acrylique vendue sous la dénomination "POLYTRAP Q5 6603".

Outre les poudres hydrophobes, la phase pulvérulente peut comprendre également des poudres hydrophiles. Les poudres hydrophiles peuvent être des charges et/ou des pigments.
Parmi les charges, on peut citer :
- les micas, qui sont des silicates d'aluminium et de potassium de compositions variées, d'origine naturelle, telle que la muscovite, la phlogopite, la lépidolite, la biotite et la séricite, ou d'origine synthétique,
- l'oxychlorure de bismuth,
- les silices, qui peuvent être sous forme de plaquettes ou de sphères telles que la silice commercialisée sous la dénomination "SILICA BEADS SB 700" par la société MYOSHI,
- les poudres de polymères hydrophiles, qui sont d'origine synthétique comme les polyacrylates, par exemple le "MICROPEARL M 100" de la société MATSUMOTO, les polyamides acryliques tels que ceux commercialisés par la société ORIS, les polyuréthanes tels que le "PLASTIC POWDER D 800" de la société TOSHNU ou qui sont d'origine naturelle, comme les drivés de cellulose ou d'amidon, par exemple les microsphères poreuses de cellulose,
- le kaolin, qui est un silicate d'aluminium hydraté,
- l'hydroxyapatite,
- les oxydes de zinc ou de titane,
- le carbonate de calcium,
- le carbonate et hydrocarbonate de magnésium.
Les charges traitées hydrophiles peuvent être des matières pulvérulentes traitées par enrobage ou greffage chimique, pour rendre leur surface hydrophile, à l'aide de matières, telles que le chitosane, le dioxyde de titane, la silice ou des polymères hydrophiles, notamment des polyesters sulfoniques ou des polyammoniums quaternaires.

Les pigments peuvent être tout pigment coloré hydrophile. Ces pigments peuvent être des pigments minéraux, organiques ou des pigments nacrés, enrobés ou non. Parmi les pigments, on peut citer les pigments minéraux tels que le dioxyde de titane (rutile ou anatase) éventuellement traité en surface, les oxydes de fer noir, jaune, rouge, le violet de manganèse, le bleu outremer, le violet d'outremer, l'oxyde de chrome anhydre ou hydraté et le bleu ferrique. Les pigments organiques peuvent être choisis parmi le noir de carbone, les pigments de type D & C, et les laques à base de carmin de cochenille.
Les nacres peuvent être choisies parmi les pigments nacrés tels que le mica recouvert de pigments organiques et/ou minéraux tel que l'oxyde de titane ou l'oxychlorure de bismuth, le mica titane recouvert de pigments organiques et/ou minéraux tel que les oxydes de fer, le bleu ferrique ou l'oxyde de chrome, ainsi que les pigments nacrés à base d'oxychlorure de bismuth.

La quantité de matière pulvérulente hydrophile introduite dans le mélange pulvérulent peut aller de 5 % à 75 % en poids par rapport au poids total du mélange pulvérulent, et de préférence de 25 % à 60 %.

Avantageusement, le rapport pondéral de la quantité de matière pulvérulente hydrophile par rapport à la quantité de matière hydrophobe peut aller de 0,08 à 7,5, et de préférence de 0,40 à 3,25.

La phase aqueuse contient obligatoirement de l'eau en quantité suffisante pour hydrater le plâtre en sulfate de calcium dihydraté. Elle peut comprendre éventuellement au moins un additif soluble ou dispersible dans l'eau. Comme additifs on peut citer des polymères hydrosolubles ou hydrodispersibles, des tensioactifs, des cires. Des actifs cosmétiques peuvent aussi être ajoutés dans la phase aqueuse comme par exemple des agents hydratants tels que la glycérine, le propylène glycol, ou bien encore des agents antioxydants, des filtres solaires.

La phase aqueuse peut être mélangée au mélange pulvérulent dans une proportion pondérale mélange pulvérulent/phase aqueuse allant de 0,2 à 2 et de préférence de 0,5 à 1,5 de façon à obtenir un mélange coulable.

Lorsque la pâte obtenue par mélange du mélange pulvérulent et de la phase aqueuse est coulée dans un moule, on laisse durcir le mélange à température ambiante. Pour accélérer le temps de prise, il est possible de laisser sécher la pâte en plaçant les moules dans une enceinte chauffée à une température pouvant aller par exemple jusqu'à 25 °C. Lorsque la composition finale est bien sèche, elle peut être directement utilisée dans son moule. On peut aussi démouler la composition et placer celle-ci dans un conditionnement approprié.

On va maintenant donner des exemples illustrant la présente invention sans toutefois la limiter.

### Exemple 1 : (invention)

On a préparé un système conservateur (A) par mélange de 17, 73 g de para hydroxybenzoate de méthyle et de 82,27 g de monolaurate de sorbitane polyoxyéthyléné à 20 moles d'oxyde d'éthylène ("TWEEN 20 de ICI).

Ce système conservateur a une viscosité de 0,58 Pa.s, mesurée à 24,9 °C sur un viscosimètre Rhéomat RM115 de chez CONTRAVES, au mobile MSDIN 114, au taux de cisaillement 221,3 s⁻¹.

On a ensuite préparé un mélange pulvérulent ayant la formulation suivante :
- Sulfate de calcium hémihydraté 25 g
- Poudres hydrophiles 29,8 g
- Poudres hydrophobes 33,7 g
- Corps gras 6 g
- Pigments 3,4 g
- Système conservateur (A) 2,1 g

On a ajouté au mélange pulvérulent 125 g d'eau puis malaxé le mélange jusqu'à obtention d'une pâte coulable. La pâte a ensuite versé dans des coupelles puis séchée.

Le produit obtenu après séchage se présente sous forme de pain et se délite facilement à l'aide d'une houpette.

On a mesuré la teneur en para hydroxybenzoate de méthyle en deux zones distinctes de la composition sèche. Pour cela, on a prélevé une partie de la composition en surface du pain et une partie dans le fond du pain. Pour chaque partie prélevée, on a extrait de manière connue pour l'homme de métier de la chimie analytique le conservateur et dosé celui-ci par les techniques classiques de chromatographie en phase liquide.

On a obtenu les résultats de dosage suivant:
concentration du para hydroxybenzoate en surface : 0,29 %
concentration du para hydroxybenzoate dans le fond : 0,27 %

On constate donc que la concentration du conservateur est sensiblement identique dans les deux zones du pain analysées. La répartition du conservateur dans la composition obtenue est donc homogène.

### Exemple 2 : (invention)

On a préparé une composition de façon similaire à celle de l'exemple 1, a la différence que le système conservateur (A) a été mélangé avec de la poudre de nylon avant d'être ajouté dans le mélange pulvérulent.

Pour cela, on a donc mélangé une préparation pulvérulente P1 comprenant 4,94 g de para hydroxy benzoate de méthyle, 22,92 g de Tween 20 et 72,14 g de poudre de nylon (ORGASOL 2002 ED NAT COS).

On a ensuite préparé un mélange pulvérulent ayant la formulation suivante :
- Sulfate de calcium hémihydraté 25 g
- Poudres hydrophiles 29,8 g
- Poudres hydrophobes 28,3 g
- Corps gras siliconés 6 g
- Pigments 3,4 g
- Préparation pulvérulente P1 7,5 g

On a ajouté au mélange pulvérulent 125 g d'eau puis malaxé le mélange jusqu'à obtention d'une pâte coulable. La pâte a ensuite versé dans des coupelles puis séchée.

Le produit obtenu après séchage se présente sous forme de pain et se délite facilement à l'aide d'une houpette.

On a obtenu les concentrations suivantes en conservateur:
surface du pain : 0, 26 %
fond du pain : 0, 26 %

On obtient ainsi une composition ayant une concentration homogène en conservateur.

### Exemple 3 : (comparatif)

On a préparé une composition sensiblement similaire à celle de l'exemple 1 mais le conservateur a été introduit directement dans le mélange pulvérulent.

Le mélange pulvérulent préparé avait la composition suivante :
- Sulfate de calcium hémihydraté 25 g
- Poudres hydrophiles 27,4 g
- Poudres hydrophobes 29,52 g
- Corps gras siliconés 6 g
- Pigments 3,4 g
- Parfum 0,21 g
- Tween 20 1 g
- para hydroxy benzoate de méthyle (poudre) 0,37 g

On a ajouté au mélange pulvérulent 125 g d'eau puis malaxé le mélange jusqu'à obtention d'une pâte coulable. La pâte a ensuite versé dans des coupelles puis séchée.

Le produit obtenu après séchage se présente sous forme de pain.

On a obtenu les concentrations suivantes en conservateur :
surface du pain : 0, 22 %
fond du pain : 0, 58 %

On obtient ainsi une composition ayant une concentration en conservateur plus élevée dans le fond de la composition et plus faible en surface de la composition. La répartition du conservateur dans la composition n'est donc pas sensiblement identique.

### Exemple 4 : (comparatif)

On a préparé une composition sensiblement similaire à celle de l'exemple 1 mais le conservateur a été introduit directement dans la phase aqueuse.

Le mélange pulvérulent préparé avait la composition suivante :
- Sulfate de calcium hémihydraté 25 g
- Poudres hydrophiles 27,4 g
- Poudres hydrophobes 29,89 g
- Corps gras siliconés 6 g
- Pigments 3,4 g
- Parfum 0,21 g
- Tween 20 1 g

On a ajouté au mélange pulvérulent une phase aqueuse comprenant 42,38 g d'eau déminéralisée et 0,21 g de para hydroxy benzoate de méthyle.

Après séchage, on a obtenu un pain dans lequel la concentration en conservateur est plus élevée dans le fond et plus faible à la surface de la composition.

### Exemple 5 : (invention)

En procédant de la même façon qu'à l'exemple 1, on a préparé un système conservateur (B) par mélange de 40 g de conservateur "LIQUAPAR OIL" et 60 g de "Tween 20". Ce système conservateur a une viscosité de 0,77 Pa.s, mesurée à 25,3 °C, sur un viscosimètre Rhéomat RM115 de chez CONTRAVES, au mobile MSDIN 108, au taux de cisaillement 221,3 s⁻¹. Puis on a préparé le mélange pulvérulent ayant la composition suivante :
- Sulfate de calcium hémihydraté 25 g
- Poudres hydrophiles 29,8 g
- Poudres hydrophobes 34,87 g
- Corps gras siliconés 6 g
- Pigments 3,4 g
- Système conservateur (B) 0,93 g

On a ajouté au mélange pulvérulent 125 g d'eau puis malaxé le mélange jusqu'à obtention d'une pâte coulable. La pâte a ensuite versé dans des coupelles puis séchée.

Le produit obtenu après séchage se présente sous forme de pain .

On a obtenu les concentrations suivantes en conservateur:
surface du pain : 0, 35 %
fond du pain : 0, 34 %

On obtient ainsi une composition ayant une concentration sensiblement constante en conservateur.

### Exemple 6 :

On a préparé une composition sensiblement identique à celle de l'exemple 5, mais en changeant le solvant du système conservateur.
On a ainsi préparé un système conservateur (C) par mélange de 26,62 g de "LIQUAPAR OIL" et de 73,38 g de palmitate d'isopropyle. Ce système conservateur a une viscosité de 0,023 Pa.s, mesurée à 25,8 °C, sur un viscosimètre Rhéomat RM115 de chez CONTRAVES, au mobile MSDIN 125, au taux de cisaillement 221,3 s⁻¹. Puis on a préparé le mélange pulvérulent ayant la composition suivante :
- Sulfate de calcium hémihydraté 25 g
- Poudres hydrophiles 29,8 g
- Poudres hydrophobes 34,41 g
- Corps gras 6 g
- Pigments 3,4 g
- Système conservateur (C) 1,39 g

Le produit obtenu après séchage se présente sous forme de pain et se délite facilement à l'aide d'une houpette.
On a obtenu les concentrations suivantes en conservateur :
surface du pain : 0, 34 %
fond du pain : 0, 37 %

On obtient ainsi une composition ayant une concentration homogène en conservateur.

### Exemple 7 : (invention)

On a préparé une composition similaire à celle de l'exemple 2 mais en utilisant le "LIQUAPAR OIL" comme conservateur.

La viscosité du système conservateur, mesurée dans les mêmes conditions que dans l'exemple 1, est inférieure à 0,77 Pa.s.

On a obtenu un pain ayant les concentrations suivantes en conservateur :
surface du pain : 0, 34 %
fond du pain : 0, 35 %

On obtient ainsi une composition ayant une concentration homogène en conservateur.

### Exemple 8 : (comparatif)

On a préparé une composition de la même façon qu'à l'exemple 2 mais en utilisant la chlorphénésine qui est un conservateur ayant une solubilité dans l'eau de 0,6 % à 25 °C.
Pour cela, on a donc mélangé une préparation pulvérulente P2 comprenant 6,4 g de chlorphénésine, 20,3 g de Tween 20 et 73,3 g de poudre de nylon (ORGASOL 2002 ED NAT COS).

On a ensuite préparé un mélange pulvérulent ayant la formulation suivante :
- Sulfate de calcium hémihydraté 25 g
- Poudres hydrophiles 27,4 g
- Poudres hydrophobes 26,1 g
- Corps gras siliconés 6 g
- Pigments 10,5 g
- Préparation pulvérulente P2 5 g
On a ajouté au mélange pulvérulent 125 g d'eau puis malaxé le mélange jusqu'à obtention d'une pâte coulable. La pâte a ensuite versé dans des coupelles puis séchée.
Le produit obtenu après séchage se présente sous forme de pain.

On a obtenu les concentrations suivantes en conservateur:
surface du pain : 0, 219 %
fond du pain : 0, 588 %

On a ainsi constaté qu'avec un conservateur ayant une solubilité dans l'eau supérieure à 0,5 % à 25 °C, on obtient une composition ayant une concentration en conservateur plus élevée dans le fond de la composition et plus faible en surface de la composition. La répartition du conservateur dans la composition n'est donc pas sensiblement identique.

### Exemple 9 : (invention)

On a préparé un système conservateur (D) par mélange de "Tween 20" et de "Liquapar Oil" puis on a mélangé ce système conservateur avec de la poudre de copolymère acrylique. La viscosité du système conservateur, mesurée dans les mêmes conditions qu'à l'exemple 1, est inférieure à 0,77 Pa.s.

Pour cela, on a donc mélangé une préparation pulvérulente P₃ comprenant 4,94 g de "Liquapar Oil", 13,54 g de "Tween 20" et 81,52 g de poudre de copolymère acrylique ("Polytrap Q5 6603").

On a ensuite préparé un mélange pulvérulent ayant la formulation suivante :
- Sulfate de calcium hémihydraté 25 g
- Poudres hydrophiles 29,3 g
- Poudres hydrophobes 28,3 g
- Corps gras siliconés 6 g
- Pigments 3,4 g
- Préparation pulvérulente P₃ 7,5 g

On a ajouté au mélange pulvérulent 125 g d'eau puis malaxé le mélange jusqu'à obtention d'une pâte coulable. La pâte a ensuite versé dans des coupelles puis séchée.

Le produit obtenu après séchage se présente sous forme de pain et se délite facilement à l'aide d'une houpette.
On a obtenu les concentrations suivantes en conservateur :
surface du pain : 0,34 %
fond du pain : 0,30 %

On obtient ainsi une composition ayant une concentration homogène en conservateur.

## Revendications

1. Procédé de préparation d'une composition cosmétique solide à base de plâtre comprenant les étapes suivantes :
- on prépare un mélange pulvérulent comprenant au moins du sulfate de calcium hémihydraté sous forme de poudre,
- on ajoute au mélange pulvérulent un phase aqueuse,
- on malaxe le mélange pulvérulent et la phase aqueuse de façon à obtenir une mélange coulable,
- on verse le mélange coulable dans un moule,
- on laisse durcir le mélange par hydratation du sulfate de calcium hémihydraté en sulfate de calcium dihydraté,
caractérisé par le fait que l'on ajoute dans le mélange pulvérulent, avant d'ajouter la phase aqueuse, un système conservateur liquide non aqueux comprenant au moins un conservateur ayant une solubilité dans l'eau inférieure ou égale à 0,5 % à 25 °C, ledit système conservateur liquide ayant une viscosité inférieure ou égale à 4,5 Pa.s à 25 °C.

2. Procédé selon la revendication 1, caractérisé par le fait que le système conservateur liquide a une viscosité inférieure ou égale à 3 Pa.s à 25 °C.

3. Procédé selon la revendication 1 ou 2 caractérisé par le fait que le système conservateur consiste en un ou plusieurs conservateur(s).

4. Procédé selon la revendication 1 ou 2, caractérisé par le fait que le système conservateur consiste en un mélange d'un ou de plusieurs conservateur(s) et d'un solvant liquide non aqueux.

5. Procédé selon la revendication 4, caractérisé par le fait que le mélange d'un ou de plusieurs conservateurs est solubilisé, en totalité ou en partie, dans le solvant.

6. Procédé selon l'une quelconque des revendications 1 à 5, caractérisé par le fait que le conservateur est un mélange de para hydroxy benzoate d'isopropyle, de para hydroxy benzoate d'isobutyle et de para hydroxy benzoate de n-butyle.

7. Procédé selon l'une quelconque des revendications 1 à 5, caractérisé par le fait que le conservateur est le para hydroxy benzoate de méthyle.

8. Procédé selon l'une quelconque des revendications précédentes, caractérisé par le fait que le conservateur est présent dans le mélange pulvérulent en une teneur allant de 0,01 % à 2 % en poids, par rapport au poids total du mélange pulvérulent.

9. Procédé selon l'une quelconque des revendications précédentes, caractérisé par le fait que l'on ajoute au mélange pulvérulent au moins un corps gras.

10. Procédé selon la revendication 9, caractérisé par le fait que le corps gras est choisi parmi les huiles minérales, les huiles végétales, les huiles d'origine animale et les huiles de synthèse.

11. Procédé selon la revendication 9 ou 10, caractérisé par le fait que le corps gras est choisi parmi les poly alpha oléfines hydrogénées ou non, les huiles de silicone phénylées, les esters d'acides gras et d'alcool en C₁-C₈.

12. Procédé selon l'une des revendications 9 à 11, caractérisé par le fait que le corps gras est le palmitate d'isopropyle.

13. Procédé selon l'une des revendications 9 à 12, caractérisé par le fait que le corps gras est présent dans le mélange pulvérulent en un teneur allant de 0,1 % à 20 % en poids par rapport au poids total du mélange pulvérulent.

14. Procédé selon l'une quelconque des revendications précédentes, caractérisé par le fait que le solvant du système conservateur est un corps gras selon l'une des revendications 9 à 13.

15. Procédé selon l'une quelconque des revendications précédentes, caractérisé par le fait que le mélange pulvérulent comprend au moins une matière pulvérulente hydrophobe.

16. Procédé selon la revendication 15, caractérisé par le fait que la matière pulvérulente hydrophobe est une poudre traitée par enrobage ou greffage chimique choisie dans le groupe formé par les poudres traitées par des silicones, des lipoaminoacides, des savons métalliques, des dérivés fluorés, des huiles Iminérales, de la lécithine, du triisostéaroyle titanate d'isopropyle, du polyéthylène, du collagène et ses dérivés.

17. Procédé selon la revendication 15, caractérisée par la fait que la matière pulvérulente hydrophobe est une poudre hydrophobe par nature choisie dans le groupe formé par le talc, les poudres de polymères hydrophobes, les lipoaminoacides, le nitrure de bore et les savons métalliques d'acides carboxyliques en C₈-C₂₂.

18. Procédé selon la revendication 16, caractérisé par le fait que les poudres de polymères hydrophobe sont choisies dans le groupe formé par les poudres de polyamide, de polyéthylène, les microsphères expansées en matériau thermoplastique, les poudres polyfluorées, les poudres de silicone, les poudres de copolymère acrylique et les poudres de polystyrène.

19. Procédé selon l'une quelconque des revendications 15 à 18, caractérisé par le fait que la teneur en matière pulvérulente hydrophobe dans le mélange pulvérulent va de 10 % à 60 %, et de préférence de 20 % à 45 % en poids, par rapport au poids total du mélange pulvérulent.

20. Procédé selon l'une des revendications 15 à 19, caractérisé par le fait que le système conservateur est mélangé avec la matière pulvérulente hydrophobe avant d'être ajoutée au mélange pulvérulent.

21. Procédé selon la revendication 20, caractérisé par le fait que la matière pulvérulente hydrophobe est choisie dans le groupe formé par la poudre de nylon et les poudres de copolymères acryliques.

22. Procédé selon l'une quelconque des revendications précédentes, caractérisé par le fait que le mélange pulvérulent comprend une matière pulvérulente hydrophile.

23. Procédé selon la revendication 22, caractérisé par le fait que la matière pulvérulente hydrophile est choisie parmi les pigments et les charges.

24. Procédé selon la revendication 22 ou 23, caractérisé par le fait que la matière pulvérulente est présente dans le mélange pulvérulent en une teneur allant de 5 % à 75 %, de préférence de 25 % à 60 % en poids par rapport au poids total du mélange pulvérulent.

25. Procédé selon l'une des revendications 22 à 24, pris en combinaison avec l'une des revendications 13 à 17, caractérisé par le fait que le rapport pondéral de la quantité de matière pulvérulente hydrophile par rapport à la quantité de matière hydrophobe va de 0,08 à 7,5, et de préférence de 0,40 à 3,25.

26. Procédé selon l'une quelconque des revendications précédentes, caractérisé par le fait que le mélange pulvérulent comprend au moins un agent tensioactif.

27. Procédé selon la revendication 26, caractérisé par le fait que la teneur en agent tensioactif dans le mélange pulvérulent va de 0,1 % à 10 % en poids par rapport au poids du mélange pulvérulent.

28. Procédé selon l'une quelconque des revendications précédentes, caractérisé par le fait que le solvant est un tensioactif selon la revendication 26 ou 27.

29. Procédé selon l'une des revendications 26 à 28, caractérisé par le fait que le tensioactif est un ester de sorbitane polyoxyéthyléné.

30. Procédé selon l'une quelconque des revendications précédentes, caractérisé par le fait que le plâtre est présent dans le mélange pulvérulent en une teneur allant de 10 % à 70 % en poids par rapport au poids total du mélange pulvérulent, et de préférence de 15 % à 35 %.

31. Procédé selon l'une quelconque des revendications précédentes, caractérisé par le fait que l'on malaxe le mélange pulvérulent et la phase aqueuse dans une proportion pondérale allant de 0,2 à 2, de préférence de 0,5 à 1,5.

32. Composition susceptible d'être obtenue par le procédé selon l'une quelconque des revendications 1 à 31.

33. Composition cosmétique solide à base de plâtre comprenant un système conservateur réparti de façon homogène dans ladite composition, ledit système conservateur étant, hors composition, sous la forme d'un liquide non aqueux comprenant au moins un conservateur ayant une solubilité dans l'eau inférieure ou égale à 0,5 % à 25 °C, ledit système conservateur liquide ayant une viscosité inférieure ou égale à 4,5 Pa.s à 25 °C.
